# NEUE EUROPÄISCHE PATENTSCHRIFT

(11) **EP 0 854 149 B2**
(45) Veröffentlichungstag und Bekanntmachung des Hinweises auf die Entscheidung über den Einspruch: **12.11.2014**
(45) Hinweis auf die Patenterteilung: 02.05.2002
(21) Anmeldenummer: 97123040.4
(22) Anmeldetag: 31.12.1997
(51) Int. Cl.: C07H 1/00

(54) **Verfahren zur Hydrierung von Zuckern mittels eines Schalenkatalysators**
Process for the hydrogenation of sugars using a shell type catalyst
Procédé d'hydrogénation de sucres avec un catalyseur en coquille

(30) Priorität: 16.05.1997 DE 19720496; 17.01.1997 DE 19701440
(43) Veröffentlichungstag der Anmeldung: 22.07.1998
(73) Patentinhaber: Südzucker Aktiengesellschaft Mannheim/Ochsenfurt, 68165 Mannheim (DE)
(72) Erfinder: Degelmann, Hanspeter, 67549 Worms (DE); Kowalczyk, Jörg, Dr., 67248 Bockenheim (DE); Kunz, Markwart, Dr., 67550 Worms (DE); Schüttenhelm, Matthias, Dr., 67550 Worms (DE)
(74) Vertreter: Schrell, Andreas

(56) Entgegenhaltungen:
- EP-A1- 0 423 525
- EP-A1- 0 625 578
- EP-B- 0 625 578
- DE-A- 2 362 552
- DE-A- 4 335 360
- DE-A1- 4 335 360
- DE-A1- 19 523 008
- GB-A- 430 576
- GB-A- 801 732
- GB-A- 989 532

## Beschreibung

Die vorliegende Erfindung betrifft ein Verfahren zur Hydrierung von Isomaltulose zu einem Zuckeralkoholgemisch, wobei die Isomaltulose in wässriger Lösung unter erhöhter Temperatur und erhöhtem Druck mit Wasserstoff unter Verwendung eines Katalysators hydriert wird.

Aus der GB 989,532 A ist ein Verfahren zur Hydrierung von Lactose unter Verwendung von Raney-Nickel zur Gewinnung von Lactit bekannt. Ebenfalls ist die Hydrierung von Lactulose beschrieben.

Die GB 430,573 A beschreibt ein Verfahren zur Herstellung von Glycerin und Glycolen aus Polyolen, wobei beispielsweise ein Zuckeralkohol mit Wasserstoff bei einer Temperatur von 200°C bis 300°C unter einem Druck von mindestens 70 Atmosphären in Gegenwart eines Katalysators, beispielsweise eines Nickel-Katalysators hydriert wird.

Die GB 801,732 A beschreibt ein Verfahren zur Herstellung eines Polyols, wobei ein entsprechender Zucker unter Druck in Gegenwart einer wässrigen Lösung eines oder mehrerer aliphatischer Alkohole mit 1 bis 3 Kohlenstoffatomen und eines Nickel-Katalysators hydriert wird. Die Konzentration des aliphatischen Alkohols in der wässrigen Lösung beträgt etwa 70 bis 95 Gew.-%.

Darüber hinaus ist aus der DE 2 362 552 A ein Verfahren zur katalytischen Hydrierung von Zuckern bei verbesserter Mannitausbeute bekannt. Als Zucker werden Invertzucker, Fructose, Glucose und Saccharose eingesetzt. Die Hydrierung wird in Gegenwart eines Hydrierungskatalysators und eines Zusatzes, beispielsweise einer Zinkverbindung oder einer Magnesiumverbindung durchgeführt.

Aus der EP 0 152 779 B1 ist ein Verfahren zur Herstellung eines Gemisches von 1-0-α-D-Glucopyranosyl-D-mannit (im folgenden 1,1 - GPM genannt) und 6-0-α-D-Glucopyranosyl-D-sorbit (im folgenden 1,6-GPS genannt) aus 6-0-α-D-Gluco-pyranosyl-D-fructose (Isomaltulose, Palatiriose^{R}) bekannt. Gemäß dem beschriebenen Verfahren wird Isomaltulose bei erhöhtem Druck und erhöhter Temperatur kontinuierlich im Festbettverfahren hydriert, wobei Katalysatoren aus Elementen der achten Nebengruppe des Periodensystems, insbesondere Nickel, Kobalt und Eisen, verwendet werden. Das Produkt der beschriebenen Verfahrensweise weist 1,6-GPS und 1,1-GPM in einem Verhältnis von ca. 1:1 auf.

Aus der DE 44 16 115 A1 ist ebenfalls ein Verfahren zur Herstellung von 1,6-GPS und 1,1-GPM aus Isomaltulose bekannt, wobei der in der EP 0 152 779 B1 beschriebene Katalysator zusätzlich Elemente der sechsten Nebengruppe des Periodensystems enthält. Auch in diesem Fall wurde eine Produktzusammensetzung von 1,6-GPS zu 1,1-GPM von ca. 1:1 erhalten. Die DE 44 16 408 A1 und die DE 39 34 457 A1 beschreiben ebenfalls Verfahren zur Hydrierung von Zuckern, beispielsweise von Glucose, Xylose, Lactulose oder Maltose. Die für die Hydrierungsreaktion verwendeten Katalysatoren sind trägerfreie Formkörper aus Elementen der achten und sechsten Nebengruppe des Periodensystems.

In Fällen, in denen bei der Hydrierung aus einem Edukt, wie zum Beispiel Isomaltulose, stereoisomere Produkte entstehen können, ist es häufig aus verfahrens- und anwendungstechnischen Gründen wünschenswert, die Stereoselektivität der Reaktion so einzustellen, daß ein bestimmtes Verhältnis der Produkte zueinander erhalten wird. Die Entwicklung von Verfahren, die zu einer bestimmten, bisher nicht erhältlichen Produktzusammensetzung führen, ist daher wünschenswert. Zudem sind die bekannten Verfahren hinsichtlich ihrer Verfahrensführung, der Handhabbarkeit der verwendeten Katalysatoren und der Verfahrenskosten verbesserungsfähig.

Das der vorliegenden Erfndung zugrundeliegende technische Problem besteht also darin, ein Verfahren zur Hydrierung von Zuckern zu Zuckeralkoholen bereitzustellen, das die vorgenannten Nachteile überwindet und/oder im Fall stereoisomerer Hydrierungsprodukte zu bisher nicht erhältlichen Produktzusammensetzungen führt.

Das der Erfindung zugrundeliegende technische Problem wird durch die Bereitstellung eines Verfahrens gemäß Hauptanspruch gelöst. Die Erfindung sieht also insbesondere vor, ein Verfahren zur Hydrierung von Isomaltulose zu einem Zuckeralkoholgemisch bereitzustellen, wobei die Isomaltulose in wässriger Lösung unter erhöhter Temperatur von 60°C bis 150°C und erhöhtem Druck von 50 bar bis 450 bar mit Wasserstoff unter Verwendung eines ein Gemisch aus einem reinen Raney-Metall, nämlich Nickel, und einer Raney-Metallegierung, nämlich einer Nickel-/Aluminiumlegierung, enthaltenden Schalen katalysators kontinuierlich im Festbettverfahren hydriert werden und wobei der Schalenkatalysator einen katalytisch weitgehend inaktiven und als Träger wirkenden Kern sowie eine katalytisch aktive Schale aufweist und wobei die Zusammensetzung stereoisomere Produkte enthaltender Produktzusammensetzungen gezielt gesteuert wird, indem das stöchiometrische Verhältnis des zur Hydrierung eingesetzten Wasserstoffs zum Edukt verändert wird.

In besonders vorteilhafter Weise ermöglicht es die erfindungsgemäße Vorgehensweise in Fällen, in denen aus einem Edukt unterschiedliche stereoisomere Produkte aus der Hydrierungsreaktion hervorgehen, wie es bei Ketosen der Fall ist, eine von der herkömmlicherweise erhaltenen Produktzusammensetzung abweichende Zusammensetzung zu erhalten.

Das erfindungsgemäße Verfahren führt bei der Hydrierung der Isomaltulose, da es sich um ein Fructose-Derivat handelt, zu einem vergleichsweise erhöhten Anteil des Sorbit-Epimers und einem verminderten Anteil des Mannit-Epimers im Produkt. Die Erfindung sieht also vor, Isomaltulose zu hydrieren, wobei sich eine von der zu erwartenden Produktzusammensetzung von ca. 50 Gew.-% 1,1-GPM zu 50 Gew.-% 1,6-GPS abweichende Zusammensetzung ergibt, insbesondere in einer bevorzugten Ausführung eine Zusammensetzung mit einem verminderten 1,1-GPM- (Mannit-Epimer) und einem erhöhten 1,6-GPS-(Sorbit-Epimer)Anteil im Produkt.

Als Mannit- und Sorbit-Epimeren werden die Zukkeralkohol-Stereoisomeren bezeichnet, die bei der Hydrierung des prochiralen Carbonyl-Kohlenstoffatoms der Ketose beziehungsweise Fructose oder glycosylsubstituierten Fructose oder Ketose (Isomaltulose, Trehalulose usw.) entstehen. Im Zusammenhang mit der vorliegenden Erfindung wird unter einem Mannit-Epimer ein Epimer verstanden, das die Konstitution der Polyolkette der, gegebenenfalls glycosylsubstituierten, D-Mannose aufweist. Unter einem Sorbit-Epimer wird ein Epimer verstanden, das die Konstitution der Polyolkette der, gegebenenfalls glycosylsubstituierten, D-Glucose aufweist.

Der erfindungsgemäß einzusetzende Schalenkatalysator enthält eine Raney-Metallegierung und ein reines Raney-Metall, wobei letzteres als Binder für die Raney-Metallegierung dient. Der Katalysator wird hergestellt, indem eine Raney-Metallegierung mit einem reinen Raney-Metall, beispielsweise als Pulver, gegebenenfalls zusammen mit Schmiermitteln, Verformungshilfsstoffen, Plastifiziermitteln und Porenbildnem homogenisiert und geformt wird, wobei für den Formbildungsprozeß Extrusion oder Verpressen in Betracht kommen können. Die Formkörper werden gegebenenfalls bei Temperaturen zwischen 80° C und 120° C getrocknet. Anschließend wird eine Calcinierung bei Temperaturen unter 850° C, vorzugsweise zwischen 500° C und 700° C, durchgeführt. Die auf diese Weise gebildeten Katalysatorvorläufer weisen eine homogene Struktur auf und werden anschließend mit Natriumhydroxidlösung, beispielsweise einer 20 %igen, 80° C warmen Natriumhydroxidlösung, zwei Stunden behandelt und aktiviert. Die Aktivierung führt zu einer 0,05 bis 1,0 mm dicken aktivierten, ausgelaugten Schale, die katalytisch aktiv ist. Der Kern des Katalysators wird nicht ausgelaugt und ist weitgehend katalytisch inaktiv, bildet gleichsam also den Träger der aktiven Schale.

Das Gewichts-Verhältnis des Raney-Metalls, nämlich Nickel, zu der auslaugbaren Legierungskomponente, nämlich Aluminium, kann zwischen 30:70 bis 70:30 liegen. Das Gewichts-Verhältnis der Raney-Metallegierung zu dem reinen Raney-Metall kann zwischen 100:20 bis 100:0,5 liegen. Dabei sollte die Partikelgröße des reinen Raney-Metalls, also des Binders, kleiner als die Partikelgröße der Metallegierung sein. In einer besonders bevorzugten Ausführungsform bestehen 99 Gew.-% des Katalysatorvorläufers aus dem reinen Raney-Metall und der Raney-Metallegierung.

Die Zusammensetzung und Herstellungsweise des erfindungsgemäß einzusetzenden Katalysators ist in der DE 43 35 360 A1 beschrieben, auf deren Inhalt, insbesondere im Hinblick auf die Katalysatorzusammensetzung und Herstellungsweise, hiermit Bezug genommen wird.

In einer besonders bevorzugten Ausführungsform der Erfindung weist der erfindungsgemäß verwendete Katalysator ein Porenvolumen bis 0,5 cm³/g, insbesondere von 0,03 bis 0,06 cm³/g auf. Die Überprüfung des Porenvolumens kann mittels Messung der Wasseraufnahmekapazität nach Deaktivierung des Katalysators mit Hydrogenperoxid erfolgen. Die Dichte des Katalysators beträgt erfindungsgemäß bevorzugt 1,3 bis 5,5 g/cm³.

Gemäß einer weiteren bevorzugten Ausführungsform ist vorgesehen, daß der Katalysator eine Druckfestigkeit von mehr als 200 N, besonders bevorzugt von mehr als 300 N, aufweist.

Die Erfindung sieht auch vor, daß die erfindungsgemäß verwendeten Katalysatoren eine BET-Oberfläche von 1 bis 50 m² g, bevorzugt 1 bis 25 m² g, aufweisen. Die Bestimmung der BET-Oberfläche kann gemäß des Verfahrens von Brunauer, Emmet und Teller (DIN 66132) durchgeführt werden.

Die erfindungsgemäß zu verwendenden Katalysatoren können dotiert sein, das heißt, sie können neben den reinen Raney-Metallen und Raney-Metallegierungen, bezogen auf den Formkörper, bis zu 20 Gew.-%, vorzugsweise 15 Gew.-%, anderer Metalle enthalten. Diese Metalle umfassen beispielsweise Chrom, Kobalt, Titan, Platin, Eisen, Tantal, Molybdän oder Ruthenium.

Die erfindungsgemäß einzusetzenden Katalysatoren können im Extrusionsverfahren hergestellt werden. Die Erfindung sieht vorzugsweise vor, dass die Katalysatoren durch Verpressen von Pulver unter hohem Druck hergestellt werden, wobei gegebenenfalls zur Verbesserung des Haftvermögens der Metallpartikel auch Graphit und/oder Klebstoff in Mengen unter 1 Gew.-%, bezogen auf das Katalysatorgewicht, eingesetzt werden können. Zusätzlich können Schmiermittel, Porenbildner, Verformungshilfsstoffe, Plastifizierer etc., die zur Formausbildung zugesetzt werden, enthalten sein. Die Katalysatoren können in Form von Kugeln, Tabletten, Granulaten, Stäbchen, jeweils mit oder ohne Bohrungen, ausgeführt sein. Selbstverständlich ist es auch möglich, die Katalysatoren als Pulverkatalysatoren, beispielsweise im Suspensionsverfahren, einzusetzen.

Das erfindungsgemäße Verfahren verwendet als Edukt Isomaltulose. Der Zucker kann flüssig oder auch als kristallines Produkt eingesetzt werden. Das Edukt wird gemäß einer besonders bevorzugten Ausführungsform der Erfindung in entmineralisiertem Wasser gelöst, wobei eine 10 bis 70 gew.-%ige, insbesondere 15 bis 50 gew.-%ige, bevorzugt 40 gew.-%ige Lösung (bezogen auf Trockensubstanz), eingestellt wird. Der pH-Wert liegt bevorzugt in einem Bereich von 3,0 bis 12,0. Der pH-Wert kann beispielsweise durch Zugabe von wasserlöslichen, basisch reagierenden Verbindungen, wie Alkalicarbonaten oder Ammoniak in wässriger Lösung, oder sauer reagierenden Verbindungen, wie Zuckersäuren, Sorbinsäure oder Zitronensäure, auf den gewünschten pH-Wert eingestellt werden.

Das erfindungsgemäße verfahren sieht vor, die Hydrierung mit reinem Wasserstoff durchzuführen, der auf einen Druck von 50 bis 450 bar, bevorzugt 150 bis 300 bar, vorkomprimiert wird. Das stöchiometrische Verhältnis von zu reduzierendem Saccharid zu Wasserstoff beträgt vorzugsweise 1 zu größer 3, besonders bevorzugt 1 zu 7. Die Hydrierung wird kontinuierlich im Festbettverfahren durchgeführt. Dabei kann in bekannter Weise ein Gleichstrom- oder ein Gegenstromverfahren eingesetzt werden.

Das erfindungsgemäße Verfahren wird vorzugsweise in einem beispielsweise als Hochdruckrohr aus Stahl ausgeführten Hydrierreaktor durchgeführt, wobei der Hydrierreaktor ganz oder teilweise mit dem trägerfreien oder trägergebundenen Katalysator gefüllt ist. Es kann auch vorgesehen sein, den Katalysator in einem Katalysatorkorb anzuordnen. Die Erfindung umfaßt selbstverständlich auch die Verwendung von Hydrierreaktoren, die beispielsweise aus verschiedenen Einzelreaktoren aufgebaut sind. In einer besonders bevorzugten Ausführungsform sieht die Erfindung vor, in dem Hydrierreaktor Rührvorrichtungen vorzusehen, mit Hilfe derer die Edukte und das Hydriergas besser miteinander in Kontakt gebracht werden können.

Die Hydrierung wird bei Temperaturen von 60° C bis 150° C, vorzugsweise von 70° C bis 120° C, vorgenommen.

Das erfindungsgemäße Verfahren führt zur Gewinnung von einem Zuckeralkoholgemisch in einer Reinheit von bis über 99 Gew.-%, bezogen auf die Trockenmasse. Es ist möglich, den Gehalt an nicht umgesetzten Zuckern oder Zuckergemischen auf Werte von 0,2 Gew.-% oder weniger zu senken.
In vorteilhafter Weise kann die Zusammensetzung stereoisomere Produkte enthaltender Produktzusammensetzungen gezielt gesteuert werden, indem die Hydrierung des Edukts unter Berücksichtigung eines einzustellenden stöchiometrischen Verhältnisses von Wasserstoff zu Edukt durchgeführt wird. Eine Erhöhung des stöchiometrischen Wasserstoff/Edukt-Verhältnisses führt in einer bevorzugten Ausführungsform zu einer Erniedrigung des Mannit-/Sorbit-Epimer-Verhältnisses, das heißt, es wird mehr Sorbit-Epimer gebildet und umgekehrt. Das Wasserstoff/Edukt-Verhältnis läßt sich über die eingesetzte Wasserstoffmenge oder den Edukt-Durchsatz steuern.

Die Erfindung wird anhand der folgenden Figuren und der dazugehörigen Ausführungsbeispiele näher erläutert. Es zeigen:
Figur 1 einen erfindungsgemäß verwendeten Hydrierreaktor in schematischer Darstellung;
Figur 2 eine graphische Darstellung der Produktbildung in Abhängigkeit von der Zeit zum Beispiel 1.
Figur 3 eine graphische Darstellung der Abhängigkeit des 1,1-GPM/1,6-GPS-Verhältnisses in Abhängigkeit vom stöchiometrischen Verhältnis Wasserstoff/Isomaltulose (Palatinose^{R}).

### Beispiel 1 (nicht erfindungsgemäß):

### Hydrierung von Isomaltulose

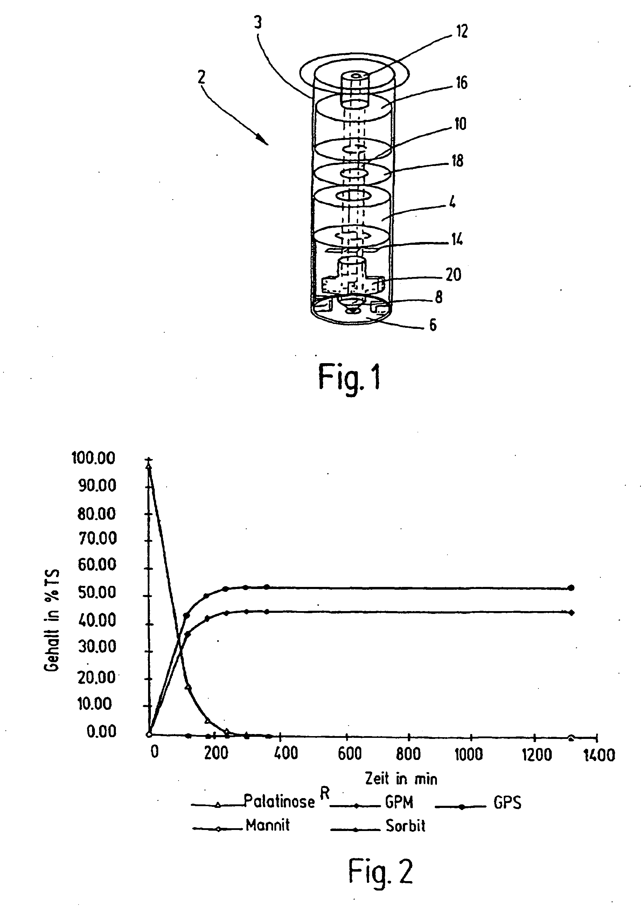

In einem 750 ml Innenvolumen aufweisenden Hydrierreaktor 2 gemäß Figur 1 wurde im diskontinuierlichen Festbettverfahren Isomaltulose hydriert. Der Hydrierreaktor 2 enthält in einem Edelstahlrohr einen Katalysatorkorb 4 mit einem Innenvolumen von 133,9 cm³.

Im Katalysatorkorb 4 befinden sich 227,4 g (feucht) Katalysator. Der Katalysator ist in Form von 4 mm-Tabletten eingesetzt worden und wurde aus einer Nikkel/Aluminiumlegierung (Ni:A1 gleich 53:47 Gew.-%) und reinem Nickel als Binder im Gewichtsverhältnis 100:15 hergestellt.

Als Porenbildner wurden 2,1 Gew.-% Wachspulver, bezogen auf das Gesamtgewicht des Katalysators, zugesetzt. Nach Homogenisierung und Zwischentrocknung wurde bei 700°C calciniert (zwei Stunden). Die Druckfestigkeit nach dem Calcinieren lag bei 280 N. Nach Aktivierung mit 20%iger Natronlauge bei 80°C für zwei Stunden wurde eine Schalendicke von 0,3 mm und eine Druckfestigkeit von >300 N erhalten. Die aktivierten Katalysatoren wurden unter Wasser gelagert und in den Experimenten der Beispiele 1 bis 12 verwendet.

Der Hydrierreaktor 2 weist an seiner im Bodenbereich angeordneten Zentrierscheibe 6 ein unteres Wellenlager 8 auf. Das untere Wellenlager 8 sowie ein oberes Wellenlager 12 dienen der Lagerung einer Edelstahl-Rührwelle 10, die Rührflügel 14 trägt. Die Rührwelle 10 wird elektromagnetisch mittels des Rührmagneten 16 angetrieben. Die Figur 1 stellt ferner Strömungsunterbrecher 18 sowie einen Gasverteilungsflügel 20 dar.

In den Hydrierreaktor 2 werden 500 ml wässrige Eduktlösung, hier Isomaltulose-Lösung (30 Gew.-% Trockensubstanz), eingefüllt, über den Gasverteilungsflügel 20 Wasserstoff mit einem Druck von 150 bar und einer Rotationsgeschwindigkeit der Rührwelle 10 von 600 Umdrehungen pro Minute eingeleitet und eine Temperatur von 70° C eingehalten. Zu Beginn der Reaktion, nach 2, 3, 4, 5, 6 und 22 Stunden werden Proben der Reaktionslösung entnommen und auf ihren Gehalt an Isomaltulose, 1,1-GPM, 1,6-GPS, Mannit, Sorbit und Restsaccharid bestimmt.

Die Ergebnisse sind in der Tabelle 1 und Figur 2 dargestellt:

**Tabelle 1**

| Proben-bez. | Fructose % | Glucose % | Isomaltulose % | Trehalulose % | Isomaltose % | Rest % |
|---|---|---|---|---|---|---|
| Ausgangslsg. | 0,1 | 0,0 | 98,3 | 1,1 | 0,3 | 0,2 |

| Versuchsdauer in min | Isomaltulose % | 1,1-GPM% | 1,6-GPS % | Mannit % | Sorbit % | Rest % |
|---|---|---|---|---|---|---|
| 0 | 98,30 | 0,00 | 0,00 | 0,00 | 0,00 | 1,70 |
| 120 | 18,05 | 36, 82 | 43,74 | 0.01 | 0,12 | 1,26 |
| 180 | 5,46 | 42,59 | 50,76 | 0,06 | 0,08 | 1,05 |
| 240 | 1,33 | 44,41 | 53,30 | 0,04 | 0,11 | 0,81 |
| 300 | 0,37 | 44,98 | 53,83 | 0,03 | 0,10 | 0,69 |
| 360 | 0,11 | 45,11 | 53,91 | 0,06 | 0,08 | 0,73 |
| 1320 | 0,05 | 45,22 | 54,02 | 0,05 | 0,09 | 0,57 |

Die Hydrierung von Isomaltulose (= Palatinose^{R}) führt zu einer von der erwarteten 1:1 Produktzusammensetzung von 1,1-GPM zu 1,6-GPS abweichenden Zusammensetzung. Das Verfahren führt zu einem erhöhten Anteil von 1,6-GPS und einem verminderten Anteil von 1,1-GPM im Produkt (siehe auch Figur 2).

### Beispiel 2:

Hydrierung von Isomaltulose bei unterschiedlichen stöchiometrischen Wasserstoff/Isomaltulose-Verhältnissen

Die Verfahrensführung und die Verfahrensapparatur entsprechen den in Beispiel 1 genannten. Das Edukt entspricht ebenfalls dem in Beispiel 1 genannten.

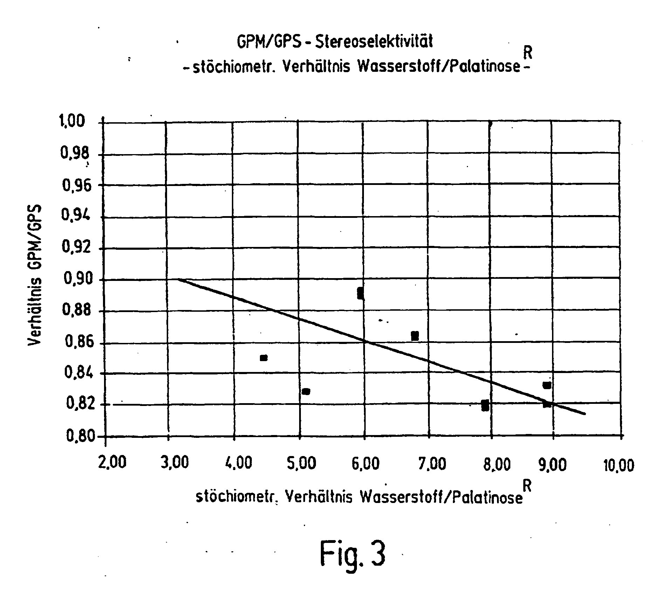

In dem vorliegenden Beispiel wurde jedoch das stöchiometrische Verhältnis des eingesetzten Wasserstoffs zum eingesetzten Edukt, das heißt der Isomaltulose, variiert, wobei -wie in Figur 3 gezeigt- das Verhältnis (Gew.-%/ Gew.-%, bezogen auf die Trockensubstanz des erhaltenen Produktgemisches) und des erhaltenen 1,1-GPM zum ebenfalls erhaltenen 1,6-GPS gezielt reguliert werden konnte. Je kleiner das stöchiometrische Verhältnis Wasserstoff/Isomaltulose desto größer wurde das Verhältnis von 1,1-GPM zu 1,6-GPS. Durch die Variation des stöchiometrischen Verhältnisses von Wasserstoff zu Isomaltulose, beispielsweise durch die Variation des Isomaltulose-Durchsatzes bei der Hydrierung, kann erfindungsgemäß gezielt die Zusammensetzung des die stereoisomeren Produkte enthaltenen Produktgemisches gesteuert werden.

## Patentansprüche

1. Verfahren zur kontinuierlichen Hydrierung von Zucker zu einem Zuckeralkoholgemisch, wobei der Zucker Isomaltulose ist, wobei der Zucker in wässriger Lösung unter einer erhöhten Temperatur von 60°C bis 150°C und einem erhöhten Druck von 50 bar bis 450 bar mit Wasserstoff unter Verwendung eines Schalenkatalysators, der ein Gemisch aus einem reinen Raney-Metall, nämlich Nickel, und einer Raney-Metalllegierung, nämlich einer Nickel-/Aluminiumlegierung, enthält, kontinuierlich im Festbettverfahren hydriert werden, wobei der Schalenkatalysator einen katalytisch weitgehend inaktiven und als Träger wirkenden Kern und eine katalytisch aktive Schale aufweist und wobei die Zusammensetzung stereoisomere Produkte enthaltender Produktzusammensetzungen gezielt gesteuert wird, indem das stöchiometrische Verhältnis des zur Hydrierung eingesetzten Wasserstoffs zum Edukt verändert wird.

2. Verfahren nach Anspruch 1, **dadurch gekennzeichnet, dass** die Druckfestigkeit des Schalenkatalysators mehr als 200 N beträgt.

3. Verfahren nach Anspruch 2, **dadurch gekennzeichnet, dass** die Druckfestigkeit des Schalenkatalysators mehr als 300 N beträgt.

4. Verfahren nach einem der vorhergehenden Ansprüche, **dadurch gekennzeichnet, dass** die Dicke der katalytisch aktiven Schale 0,05 bis 1,0 mm beträgt.

5. Verfahren nach einem der vorhergehenden Ansprüche, **dadurch gekennzeichnet, dass** das Porenvolumen des Schalenkatalysators 0,03 bis 0,06 cm³/g beträgt.

6. Verfahren nach einem der vorhergehenden Ansprüche, **dadurch gekennzeichnet, dass** die BET-Oberfläche 1 bis 50 m²/g beträgt.

7. Verfahren nach einem der vorhergehenden Ansprüche, **dadurch gekennzeichnet, dass** die erhöhte Temperatur 70° C beträgt.

8. Verfahren nach einem der vorhergehenden Ansprüche, **dadurch gekennzeichnet, dass** die Eduktkonzentration der Ausgangslösung auf 10 bis 70 Gew.-% eingestellt ist.

9. Verfahren nach Anspruch 8, **dadurch gekennzeichnet, dass** die Eduktkonzentration der Ausgangslösung auf 15 bis 50 Gew.-% eingestellt ist.

10. Verfahren nach Anspruch 8 oder 9, **dadurch gekennzeichnet, dass** die Eduktkonzentration der Ausgangslösung auf 40 Gew.-% eingestellt ist.

## Claims

1. Method of continuously hydrogenating sugar to form a sugar alcohol mixture, the sugar being isomaltulose, wherein the sugar is continuously hydrogenated by means of a fixed bed method in aqueous solution at a raised temperature of between 60°C and 150°C and a raised pressure of between 50 bar and 450 bar with hydrogen using a shell catalyst which contains a mixture of a pure Raney metal, namely nickel, and a Raney metal alloy, namely a nickel/aluminium alloy, the shell catalyst having a catalytically largely inactive core, acting as a carrier, and a catalytically active shell, wherein the composition of product compositions containing stereoisomeric products is purposefully controlled by altering the stoichiometric ratio of the hydrogen, used for hydrogenation, to the starting material.

2. Method according to claim 1, **characterised in that** the crushing strength of the shell catalyst is more than 200 N.

3. Method according to claim 2, **characterised in that** the crushing strength of the shell catalyst is more than 300 N.

4. Method according to one of the preceding claims, **characterised in that** the thickness of the catalytically active shell is between 0.05 and 1.0 mm.

5. Method according to one of the preceding claims, **characterised in that** the pore volume of the shell catalyst is between 0.03 and 0.06 cm³/g.

6. Method according to one of the preceding claims, **characterised in that** the BET surface is between 1 and 50 m²/g.

7. Method according to one of the preceding claims, **characterised in that** the raised temperature is 70°C.

8. Method according to one of the preceding claims, **characterised in that** the starting material concentration of the initial solution is set at between 10 and 70 wt-%.

9. Method according to claim 8, **characterised in that** the starting material concentration of the initial solution is set at between 15 and 50 wt-%.

10. Method according to claim 8 or 9, **characterised in that** the starting material concentration of the initial solution is set at 40 wt-%.

## Revendications

1. Procédé d'hydrogénation en continue de sucre en un mélange d'alcool de sucre, le sucre étant l'isomaltulose, dans lequel le sucre est hydrogéné en continue selon un procédé en lit fixe en solution aqueuse sous une température accrue allant de 60°C à 150°C, et sous une pression accrue allant de 50 bars à 450 bars, avec de l'hydrogène, en utilisant un catalyseur en coquille qui contient un mélange à base d'un métal de Raney pur, soit du nickel, et un alliage de métal de Raney, soit un alliage à base de nickel/l'aluminium, le catalyseur en coquille possédant un noyau agissant comme support, largement inactif d'un point de vue catalytique et une coquille catalytiquement active, et dans lequel la composition des préparations de produits contenant des produits stéréo-isomères est contrôlée d'une manière ciblée, en modifiant le rapport stoechio-métrique de l'hydrogène utilisé pour l'hydrogénation à l'éduit.

2. Procédé selon la revendication 1, **caractérisé en ce que** la résistance à la pression du catalyseur en coquille s'élève à plus de 200 N.

3. Procédé selon la revendication 2, **caractérisé en ce que** la résistance à la pression du catalyseur en coquille s'élève à plus de 300 N.

4. Procédé selon l'une des revendications précédentes, **caractérisé en ce que** l'épaisseur de la coquille active catalytiquement s'élève à 0,05 à 1,0 mm.

5. Procédé selon l'une des revendications précédentes, **caractérisé en ce que** le volume des pores du catalyseur en coquille s'élève à 0,03 à 0,06 cm3/g.

6. Procédé selon l'une des revendications précédentes, **caractérisé en ce que** la surface BET s'élève à 1 à 50 m²/g.

7. Procédé selon l'une des revendications précédentes, **caractérisé en ce que** la température accrue s'élève à 70°C.

8. Procédé selon l'une des revendications précédentes, **caractérisé en ce que** la concentration en éduit de la solution de départ est ajustée à 10 à 70 % en poids.

9. Procédé selon la revendication 8, **caractérisé en ce que** la concentration en éduit de la solution de départ est ajustée à 15 à 50 % en poids.

10. Procédé selon la revendication 8 ou la revendication 9, **caractérisé en ce que** la concentration en éduit de la solution de départ est ajustée à 40 % en poids.
